**Europäisches Patentamt**

**European Patent Office**

**Office européen des brevets**

(19)

(11) Veröffentlichungsnummer: **0 127 086**
**B1**

(12)

# EUROPÄISCHE PATENTSCHRIFT

(45) Veröffentlichungstag der Patentschrift:
**29.10.86**

(51) Int. Cl.⁴: **C 07 C 172/00**

(21) Anmeldenummer: **84105653.4**

(22) Anmeldetag: **18.05.84**

(54) Verfahren zur Reinigung von Vitamin D3 und Vitamin D2.

(30) Priorität: **26.05.83 DE 3319026**

(43) Veröffentlichungstag der Anmeldung:
**05.12.84 Patentblatt 84/49**

(45) Bekanntmachung des Hinweises auf die Patenterteilung:
**29.10.86 Patentblatt 86/44**

(84) Benannte Vertragsstaaten:
**CH DE FR GB IT LI NL**

(56) Entgegenhaltungen:
**GB - A - 370 743**
**US - A - 2 895 971**
**US - A - 3 334 118**

(73) Patentinhaber: **BASF Aktiengesellschaft,
Carl-Bosch-Strasse 38, D-6700 Ludwigshafen (DE)**

(72) Erfinder: **Fischer, Martin, Dr., Elbinger Weg 1,
D-6700 Ludwigshafen (DE)**

ACTORUM AG

## Beschreibung

Die Erfindung betrifft ein Verfahren zur Reinigung von rohem Vitamin $D_3$ durch die Kombination einer an sich bekannten Behandlung mit Dienophilen und einer Kurzwegdestillation.

Bei Bestrahlung von 7-Dehydrocholesterin oder Ergosterin erhält man nach Abtrennung des nicht umgesetzten Ausgangsstoffs das sogenannte Vitamin $D_2$- bzw. Vitamin $D_3$-Harz, das einen Vitamingehalt von ca. 63% besitzt (The Vitamins, Vol III, S. 203, 1971, W. H. Sebrell, Jr. und R.S. Harris ed.). Unter Vitamin D-Gehalt ist die Summe der Gehalte an Vitamin D und Praevitamin D zu verstehen. Daneben enthält das Vitamin D-Harz noch einige Isomere wie Tachysterin, Lumisterin und Ergosterin bzw. 7-Dehydrocholesterin.

Es hat nicht an Versuchen gefehlt, Vitamin $D_2$ und $D_3$ von den bei der Belichtung gebildeten Nebenprodukten zu trennen. So ist es z.B. möglich, den Gehalt an Vitamin $D_3$ durch Bildung von kristallinen Additionsverbindungen mit 7-Dehydrocholesterin (FR 1 378 121, US 3 367 950) oder Cholesterin (US 2 264 320) zu erhöhen. Weiterhin lassen sich kristalline Ester von Vitamin $D_2$ oder $D_3$, z.B. die Dinitrobenzoate herstellen. Solche Ester können durch Umkristallisation gereinigt und anschliessend zu hochkonzentrierten Vitamin D-Präparaten verseift werden. Schliesslich wurde auch schon rohes Vitamin D mit Maleinsäure- und Citraconsäureanhydrid umgesetzt mit dem Ziel, das Tachysterin selektiv in Diels-Alder-Addukte zu überführen, die anschliessend mit Alkali extrahiert werden können (GB 370 743; 491 653).

Alle bisher bekannten Methoden sind jedoch technisch sehr umständlich, sind mit grossen Verlusten an den teuren Vitaminen verbunden und bringen z.T. nur geringfügige Reinigungseffekte. Insbesondere die Reinigungsmethode mit Maleinsäure- bzw. Citraconsäureanhydrid hat sich nicht durchsetzen können, weil neben Tachysterin auch Vitamin $D_2$ und $D_3$ mit diesen Dienophilen reagieren und dadurch abgebaut werden. Ausserdem katalysieren Spuren Säure, die aus den Anhydriden leicht entstehen können, die Zersetzung der Vitamine.

Es bestand daher die Aufgabe, ein Verfahren vorzuschlagen, das eine Reinigung des rohen Vitamin $D_3$ oder rohen Vitamin $D_2$ mit möglichst geringem Verlust an Vitaminaktivität erlaubt. Überraschenderweise wurde nun gefunden, dass ein nennenswerter Teil der Verunreinigungen aus Vitamin $D_2$- oder $D_3$-Harz ohne Verlust an Vitamin dadurch entfernt werden kann, dass das rohe Harz, gegebenenfalls in einem Lösungsmittel, mit einem Dienophil, das keine stark sauren Gruppen oder funktionelle Gruppen enthält, die mit Alkohol oder Wasser in stark saure Gruppen übergehen können, umgesetzt und anschliessend einer Kurzwegdestillation bei vermindertem Druck unterworfen wird.

Unter Kurzwegdestillation, auch Molekulardestillation genannt, wird eine Destillationsmethode verstanden, bei der das zu destillierende Material über eine kurze Wegstrecke von einer beheizten Fläche zu einer gekühlten Fläche übergeht, wobei im vorliegenden Fall die Verweilzeit auf der beheizten Fläche möglichst kurz, z.B. bei 190 °C, weniger als 10 Minuten betragen soll.

Geeignete Vorrichtungen sind beispielsweise in «The Application of Molecular Distillation» von Hollo, Kurucz und Borodi, Verlag Akademiai Kiado, Budapest 1971, beschrieben. Insbesondere sind solche Vorrichtungen geeignet, bei denen durch Schwerkraft oder mechanisches Verteilen dünne Filme auf der Verdampfungsoberfläche erzeugt werden. Im einzelnen sind Fallfilmverdampfer mit und ohne Wischereinrichtungen zu nennen, wobei der Abstand zwischen Heiz- und Kühlfläche zweckmässig nicht grösser als die freie Weglänge der Moleküle bei den gewählten Temperatur- und Druckbedingungen sein sollte. Dies ist in der Regel ein Bereich zwischen wenigen Millimeter und einigen Zentimeter, z.B. weniger als 5 Zentimeter, vorzugsweise 0,3 bis 3 Zentimeter.

Als Dienophile kommen alle ungesättigten Verbindungen, die mit elektronenreichen Dienen wie Cyclopentadien, Butadien oder 9,10-Dimethylanthracen bereitwillig Diels-Alder-Additionen eingehen, in Betracht, jedoch mit der Massgabe, dass sie keine stark sauren funktionellen Gruppen oder keine Strukturelemente wie beispielsweise Säurechlorid- oder Anhydridgruppen enthalten, aus denen mit Alkoholen oder Wasser starke Säuren entstehen können. Dienophile sind beispielsweise eingehend beschrieben in H. Wollweber, Diels-Alder-Reaktionen, Georg-Thieme-Verlag, Stuttgart, 1972, S. 54 bis 65, 185 bis 212; sowie bei H. Sauer, Angew. Chemie 1967, 79, 76.

Dementsprechend sind beispielsweise p-Benzochinon, Maleinsäure-N-methylimid, Maleinsäure-N-butylimid, Maleinsäure-N-phenylimid, Fumarsäureester mit niederen aliphatischen Alkoholen, Maleinester mit niederen aliphatischen Alkoholen, Acrylnitril, Methacrylnitril, 1,2-Dicyanethylen, 1,1-Dicyanethylen, Acrylester mit niederen aliphatischen Alkoholen oder Diolen, Methacrylester mit niederen aliphatischen Alkoholen, Acetylendicarbonester mit niederen aliphatischen Alkoholen, Propiolester mit niederen aliphatischen Alkoholen, ungesättigte Sulfone wie 1,2-Diphenylsulfonylethylen, ungesättigte Ketone wie 1,2-Dibenzoylethylen, ungesättigte Nitroverbindungen wie β-Nitrostyrol, Azodicarbonester mit niederen aliphatischen Alkoholen, elektronenarme aromatische Azoverbindungen wie p,p'-Dinitroazobenzol und Nitrosoverbindungen wie Nitrosobenzol, zu nennen.

Um die anschliessende Kurzwegdestillation zu erleichtern, ist es zweckmässig, Dienophile auszuwählen, die deutlich leichter flüchtig als Vitamin D sind. Auf diese Weise kann ein nach der Umsetzung mit Vitamin D-Harz verbliebener Rest an nicht umgesetztem Dienophil vor der Kurzwegdestillation des Vitamins abdestilliert werden. Insbesondere kommen Acrylsäuremethylester, Acrylsäureethylester, Acrylsäure-n-butylester, Butandioldiacrylat, p-Benzochinon oder N-Methylmaleinimid als Dienophile in Betracht.

Bezogen auf ein Mol des zu reinigenden Vitamin D-Harzes werden in der Regel 0,05 bis 5 Mol, bevorzugt 0,1 bis 0,5 Mol, Dienophil eingesetzt. Die Umsetzung mit dem Dienophil kann in Gegenwart beliebiger organischer Lösungsmittel, die unter den Reaktionsbedingungen inert sind, z.B. Ether, Toluol und Heptan oder auch in Abwesenheit von Lösungsmitteln durchgeführt werden. Soll für die Kurzwegdestillation ein Träger, z.B. Erdnussöl, verwendet werden, so ist es zweckmmässig, die Umsetzung mit dem Dienophil in Gegenwart des Trägeröls durchzuführen, weil damit die hohe Viskosität von Vitamin $D_3$-Harz herabgesetzt und entsprechend die Vermischung der Reaktionspartner erleichtert wird.

Die Umsetzung mit dem Dienophil kann bei Temperaturen von 0 bis 150°C, bevorzugt bei Temperaturen von 20 bis 80°C durchgeführt werden. Je nach Reaktivität des Dienophils sind Reaktionszeiten von wenigen Minuten bis zu ca. 20 Stunden erforderlich. Ein nach der Umsetzung noch verbliebener Überschuss an nicht umgesetztem Dienophil wird bei Drücken von 0,001 bis 1 000 mbar und Temperaturen von 20 bis 120°C abdestilliert.

Das von überschüssigem Dienophil befreite Vitamin D wird schliesslich bei 130 bis 200°C und Drücken von 0,001 bis 0,1 mbar in einer kontinuierlich betriebenen Kurzwegdestillationsapparatur, bei der Verdampfungs- und Kondensationsfläche nicht mehr als 5 cm voneinander entfernt sein sollen, überdestilliert. Für die Kurzwegdestillation kann ein schwerflüchtiges Trägeröl, z.B. ein hochsiedendes Paraffin oder Ernussöl, verwendet werden. Temperatur, Druck und Zulauf werden dabei zweckmässig so eingestellt, dass 70 bis 95% des Vitamins überdestillieren. Das Destillat besitzt je nach Reinheitsgrad des eingesetzten rohen Vitamin D-Harzes Vitamin D-Gehalte zwischen 65 und 78%. Somit wird durch das erfindungsgemässe Verfahren der Vitamingehalt um 5 bis 15% (absolut) erhöht.

Der Sumpf der Kurzwegdestillation kann bis zu fünfmal rückgeführt werden. Die Verluste an Wertprodukt liegen dann insgesamt unter 3%.

*Beispiel 1*

0,4 g p-Benzochinon und 19,3 g Vitamin $D_3$-Harz, das 11,7% Praevitamin $D_3$ und 49,0% Vitamin D enthielt, wurden in 100 ml n-Heptan gelöst. Nachdem die Lösung 6 Stunden bei 25°C gestanden hatte, wurden 19,3 g Erdnussöl zugesetzt und das Heptan bei 30 mbar abdestilliert, wobei die Badtemperatur bis auf 80°C gesteigert wurde. Der Destillationsrückstand wurde anschliessend während 84 min in eine Kurzwegdestillationsapparatur kontinuierlich eindosiert. Das Vitamin $D_3$ destilliert bei 150°C/0,01 mbar über einen Abstand von 1,3 cm von der Verdampfer- zur Kondensatorfläche. Es wurden 12,8 g Destillat mit einem Gehalt von 15,9% Praevitamin $D_3$ und 57,1% Vitamin $D_3$ erhalten. Als Destillationssumpf wurden 26 g eines Öls erhalten, das 1,8% Praevitamin $D_3$ und 7,0 % Vitamin $D_3$ enthielt.

*Beispiel 2*

1,6 g p-Benzochinon und 85,4 g Vitamin $D_3$-Harz, das 12,8% Praevitamin $D_3$ und 51,4% Vitamin D enthielt, wurden bei 60°C mit 85,4 g Erdnussöl vermischt. Das Gemisch wurde 200 min bei 25°C gehalten und unter den Bedingungen von Beispiel 1 einer kontinuierlichen Kurzwegdestillation unterworfen. Man erhielt 69,8 g Destillat mit einem Gehalt von 13,7% Praevitamin $D_3$ und 59,8 % Vitamin $D_3$. 101 g Destillationssumpf enthielten 0,5% Praevitamin $D_3$ und 2,3% Vitamin $D_3$.

*Beispiel 3*

4,0 g Acrylsäuremethylester und 50 g Vitamin $D_3$-Harz, das 9,8% Praevitamin $D_3$ und 50,4%. Vitamin $D_3$ enthielt, wurden bei 60°C mit 50 g Erdnussöl gemischt. Nach 2 Stunden Erwärmen auf 60°C wurden Spuren nicht umgesetzten Acrylesters bei 80°C/30 mbar verdampft und das verbliebene Öl anschliessend wie in Beispiel 1 beschrieben einer kontinuierlichen Kurzwegdestillation unterworfen. Man erhielt 32,1 g Destillat mit einem Gehalt von 11,7% Praevitamin $D_3$ und 63,2% Vitamin $D_3$. 71,0 g Destillationssumpf enthielten 1,8% Praevitamin $D_3$ und 6,6% Vitamin $D5_3$.

*Beispiel 4*

1,2 g Acrylsäure-n-butylester und 19,9 g Vitamin $D_3$-Harz, das 7,2% Praevitamin $D_3$ und 58,2% Vitamin $D_3$ enthielt, wurden bei 60°C mit 19,9 g Erdnussöl gemischt. Nach 2 Stunden Erwärmen auf 50°C wurden Spuren nicht umgesetzten Acrylesters bei 80°C/30 mbar verdampft und das verbliebene Öl anschliessend wie in Beispiel 1 beschrieben einer kontinuierlichen Kurzwegdestillation unterworfen. Man erhielt 15,0 g Destillat mit einem Gehalt von 10,8% Praevitamin $D_3$ und 62,7% Vitamin $D_3$. 25,5 g Destillationssumpf enthielten 2,0% Praevitamin $D_3$ und 5,8% Vitamin $D_3$.

*Beispiel 5*

1,0 g N-Methylmaleinimid und 20 g Vitamin $D_3$-Harz, das 11,0% Praevitamin $D_3$ und 48,8% Vitamin $D_3$ enthielt, wurden in 100 ml Methyl-tert.-butylether gelöst. Nach 20stündigem Stehen bei 25°C wurden der Mischung 20 g Erdnussöl zugesetzt und das Lösungsmittel bei 30 mbar abdestilliert, wobei die Badtemperatur bis auf 80°C gesteigert wurde. Das verbleibende Öl wurde anschliessend wie in Beispiel 1 beschrieben einer kontinuierlichen Kurzwegdestillation unterworfen. Man erhielt 16 g Destillat mit einem Gehalt von 9,7%. Praevitamin $D_3$ und 63,4% Vitamin $D_3$. 25 g Destillationsrückstand enthielten 0,4% Praevitamin $D_3$ und 1,2% Vitamin $D_3$.

*Beispiel 6*

1,6 g N-Phenylmaleinimid und 20 g Vitamin $D_3$-Harz, das 11,9% Praevitamin und 48,8%. Vitamin $D_3$ enthielt, wurden in 100 ml Methyl-tert.-butylether gelöst. Nach den Angaben des Beispiels 5 wurden 15,5 g Destillat mit einem Gehalt von 10,2% Praevitamin $D_3$ und 61,4% Vitamin $D_3$ erhalten. 26 g Destillationsrückstand enthielten 0,5% Praevitamin $D_3$ und 3,3% Vitamin $D_3$.

*Beispiel 7*

4,8 g Acrylsäuremethylester und 50 g Vitamin $D_2$-Harz, das 8,7% Praevitamin $D_2$ und 54,2% Vitamin $D_2$ enthielt, wurden bei 60°C in 50 g Erdnussöl gelöst. Nach 2 Stunden Erwärmen auf 60°C wurden Spuren nicht umgesetzten Acrylesters bei 80°C/30 mbar verdampft und das verbliebene Öl anschliessend wie in Beispiel 1 beschrieben einer kontinuierlichen Kurzwegdestillation unterworfen. Man erhielt 35,2 g Destillat mit einem Gehalt von 9,7% Praevitamin $D_2$ und 88,1% Vitamin $D_2$. 68 g Destillationssumpf enthielten 1,1% Praevitamin $D_2$ und 4,8% Vitamin $D_2$.

## Beispiel 8

60 g Acrylsäuremethylester und 1000 g Vitamin $D_3$-Harz, das 11,2% Praevitamin $D_3$ und 52,4% Vitamin $D_3$ enthielt, wurden bei 60°C mit 1000 g Erdnussöl gemischt. Nach 2 Stunden Erwärmen auf 60°C wurden Spuren nicht umgesetzten Acrylesters bei 80°C/30 mbar verdampft und das verbliebene Öl anschliessend während 60 min in eine Kurzwegdestillationapparatur kontinuierlich eindosiert. Das Vitamin $D_3$ destilliert bei 188°C/0,01 mbar über einen Abstand von 2,5 cm von der Verdampfer- zur Kondensatorfläche. Es wurden 768 g Destillat mit einem Gehalt von 11,8% Praevitamin $D_3$ und 64,0% Vitamin $D_3$ erhalten. 1282 g Destillationsrückstand enthielten 0,5% Praevitamin $D_3$ und 3,7% Vitamin $D_3$.

Der Destillationssumpf wurde mit 1282 g Vitamin $D_3$-Harz, das 11,2% Praevitamin $D_3$ und 52,4% Vitamin $D_3$ enthielt, und mit 77 g Acrylsäuremethylester bei 60°C vermischt und das erhaltene Gemisch wie oben beschrieben verarbeitet. Es wurden 1090 g Destillat mit einem Gehalt von 9,9% Praevitamin $D_3$ und 62,1% Vitamin $D_3$ erhalten. 1540 g Destillationssumpf enthielten 0,8% Praevitamin $D_3$ und 4,6% Vitamin $D_3$.

Der Destillationssumpf wurde erneut mit 1540 g Vitamin $D_3$-Harz, das 11,2% Praevitamin $D_3$ und 52,4% Vitamin $D_3$ enthielt, und mit 92 g Acrylsäuremethylester bei 60°C vermischt und wie oben beschrieben verarbeitet. Es wurden 1335 g Destillat mit einem Gehalt von 10,1% Praevitamin $D_3$ und 60,7% Vitamin $D_3$ erhalten. 1820 g Destillationssumpf enthielten 0,7% Praevitamin $D_3$ und 5,3% Vitamin $D_3$.

## Beispiel 9

2,4 g Butandioldiacrylat und 36,3 g Vitamin $D_3$-Harz, das 11,2% Praevitamin $D_3$ und 56,3% Vitamin $D_3$ enthielt, wurden bei 60°C mit 36,3 g Erdnussöl gemischt. Nach 4 Stunden Erwärmen auf 60°C wurde die Mischung wie in Beispiel 1 beschrieben einer kontinuierlichen Kurzwegdestillation unterworfen. Man erhielt 29,2 g Destillat mit einem Gehalt von 16,7%. Praevitamin $D_3$ und 58,1% Vitamin $D_3$. 45,6 g Destillationsrückstand enthielten 1,1% Praevitamin $D_3$ und 4,8% Vitamin $D_3$.

## Beispiel 10

1,8 g Acrylnitril und 37,8 g Vitamin $D_3$-Harz, das 11,2% Praevitamin $D_3$ und 56,3% Vitamin $D_3$ enthielt, wurden bei 60°C mit 37,8% Erdnussöl gemischt. Nach 6 Stunden Erwärmen auf 60°C wurden Spuren nicht umgegetzten Acrylnitrils bei 80°C/30 mbar verdampft und das verbliebene Öl anschliessend wie in Beispiel 1 beschrieben einer kontinuierlichen Kurzwegdestillation unterworfen. Man erhielt 29,3 g Destillat mit einem Gehalt von 14,5%. Praevitamin $D_3$ und 59,4% Vitamin $D_3$. 46,8 g Destillationsrückstand enthielten 1,2% Praevitamin $D_3$ und 8,0% Vitamin $D_3$.

## Patentansprüche

1. Verfahren zur Reinigung von Vitamin $D_3$ oder $D_2$, dadurch gekennzeichnet, dass man Vitamin $D_3$-Harz oder Vitamin $D_2$-Harz mit Dienophilen umsetzt, die keine stark sauren Gruppen oder funktionelle Gruppen enthalten, die mit Alkohol oder Wasser in stark saure Gruppen übergehen können, und anschliessend einer Kurzwegdestillation unterwirft.

2. Verfahren gemäss Anspruch 1, dadurch gekennzeichnet, dass man ein durch Bestrahlen von 7-Dehydrocholesterin oder Ergosterin erhaltenes Vitamin $D_3$-Harz verwendet.

3. Verfahren gemäss Anspruch 1, dadurch gekennzeichnet, dass man die Umsetzung mit dem Dienophil in einem Lösungsmittel durchführt.

4. Verfahren gemäss Anspruch 1, dadurch gekennzeichnet, dass man Vitamin $D_3$-Harz mt einem Dienophil umsetzt, das deutlich leichter flüchtig als Vitamin $D_3$ ist.

## Claims

1. A process for the purification of vitamin $D_3$ or $D_2$, wherein vitamin $D_3$ resin or vitamin $D_2$ resin is reacted with a dienophile which does not contain any strongly acidic groups or functional groups which can react with alcohol or water to form stronly acidic groups, and the product is then subjected to a short-path distillation.

2. A process as claimed in claim 1, wherein the vitamin $D_3$ resin used has been obtained by irradiating 7-dehydrocholesterol or ergosterol.

3. A process as claimed in claim 1, wherein the reaction with the dienophile is carried out in a solvent.

4. A process as claimed in claim 1, wherein the vitamin $D_3$ resin is reacted with a dienophile which is substantially more volatile than vitamin $D_3$.

## Revendications

1. Procédé de purification de vitamine $D_3$ ou $D_2$, caractérisé en ce qu'on fait réagir une résine de vitamine $D_3$ ou une résine de vitamine $D_2$ avec des diénophiles qui ne contiennent pas de groupements fortement acides ou de groupements fonctionnels qui, avec un alcool ou de l'eau, peuvent se transformer en groupements fortement acides, puis on la soumet à une distillation moléculaire.

2. Procédé selon la revendication 1, caractérisé en ce qu'on utilise une résine de vitamine $D_3$ obtenue par irradiation de 7-déhydrocholestérol ou d'ergostérol.

3. Procédé selon la revendication 1, caractérisé en ce qu'on effectue la réaction avec le diénophile dans un solvant.

4. Procédé selon la revendication 1, caractérisé en ce qu'on fait réagir la résine de vitamine $D_3$ avec un diénophile qui est nettement plus volatil que la vitamine $D_3$.